# EUROPEAN PATENT APPLICATION

(11) **EP 1 731 149 A1**
(43) Date of publication of application: **13.12.2006**
(21) Application number: 05012270.4
(22) Date of filing: 08.06.2005
(51) Int. Cl.: A61K 31/4015, A61P 25/08

(54) **Use of 2-oxo-1-pyrrolidone derivatives for the treatment of diseases characterized by progressive myoclonic epilepsy**

(71) Applicant: UCB S.A., 1070 Bruxelles (BE)
(72) Inventor: Verdru, Peter, Marietta GA 30067 (US)
(74) Representative: Lechien, Monique

(57) **Abstract**

The present invention relates to the use of brivaracetam for the preparation of drugs effective for the prevention or treatment of progressive myoclonic epilepsies.

## Description

The present invention relates to the use of brivaracetam for the preparation of drugs effective for the prevention or treatment of progressive myoclonic epilepsy.

British patent N° 1,039,113 describes the compound 2-pyrrolidineacetamide and states that the compound can be used for therapeutic purposes. This compound is also known as piracetam.

The use of levorotatory (S)-α-ethyl-2-oxo-1-pyrrolidineacetamide, also known as levetiracetam as a protective agent for the treatment and prevention of hypoxic and ischaemic type aggressions of the central nervous system is described in the European patent application EP-A-0162 036. This compound can also be employed in the treatment of epilepsy, a therapeutic indication for which it has been demonstrated that its dextrorotatory enantiomer, (R)-(+)-α-ethyl-2-oxo-1-pyrrolidine-acetamide, is completely devoid of activity (A. J. GOWER et al., Eur. J. Pharmacol., 222, (1992), 193-203). Levetiracetam has also been described in European patent application EP-A-0 645 139 for the treatment of anxiety.

2-oxo-1-pyrrolidine derivatives, such as brivaracetam, as well as their use as pharmaceuticals are described in the international patent application having publication number WO 01/62726. The derivatives are particularly suited for treating neurological disorders.

It has now been discovered that brivaracetam possesses therapeutic properties which render them particularly useful in preventing or treating disease states characterised by progressive myoclonic epilepsies.

In one aspect, the present invention thus relates to the use of an active compound brivaracetam for the manufacture of a medicament for preventing or treating progressive myoclonic epilepsy.

In another aspect, the present invention relates to a method of preventing or treating progressive myoclonic epilepsy in a patient by administering an effective dose of an active compound brivaracetam.

The effective dose of an active compound, as described above, administered to the patient lies between 0.03-30 mg per kg body weight for brivaracetam.

Brivaracetam is described in the international patent application having the publication number WO 01/62726, as (α¹S, 4R)-α-ethyl-2-oxo-4-propyl-1-pyrrolidineacetamide) also known as (2S)-2-[(4R)-2-oxo-4-propylpyrrolidinyl]butanamide, with the following chemical structure :

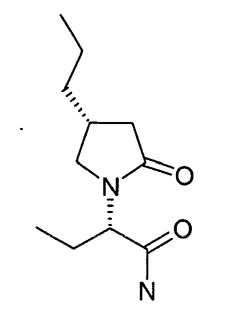

According to the invention, progressive myoclonic epilepsies (PME) characterized by myoclonic seizures, tonic-clonic seizures, and progressive neurological deterioration, typically with cerebellar signs and dementia. Progressive myoclonic epilepsies are a group of debilitating catastrophic epilepsy disorders, characterized by the occurrence of seizures, myoclonus, progressive neurological dysfunction, and an inevitable decline. The disorders have a genetic component. PME includes Unverricht-Lundborg disease, Lafora disease, Myoclonic epilepsy with ragged red fibres, neuronal ceroid lipofuscinosis, sialidoses, and dentatorubral-pallidoluysian atrophy.

Unverricht-Lundborg disease is a progressive myoclonic epilepsy. It is an autosomal recessive inherited neurodegenerative disorder caused by mutations in the cystatin B gene. In another aspect, the present invention relates to the use of an active compound brivaracetam for the manufacture of a medicament for preventing or treating disease states characterised by Unverncht-Lundborg disease.

Myoclonus in PME is typically fragmentary, and is often precipitated by posture, action, or external stimuli such as light, sound or touch. The myoclonus in PMEs tends to be multifocal, of variable amplitude with many small jerks, relatively constant, and increased by voluntary movement, that may resemble chorea. Myoclonus in these indications may also occur generalized or as a bilateral massive myoclonus. In addition, the most defining characteristic of PME are severe progressive neurological deficits, primarily ataxia and/or dementia.

Several Electroencephalogram (EEG) features are useful in distinguishing PME from epilepsies. Each clinical myoclonic event may or may not be associated with EEG spikes or spike-and-wave discharges. Unlike Juvenile myoclonic epilepsy (JME), most of the myoclonic movements in PME syndromes especially the almost continual, small-amplitude jerks, are not time-locked to EEG discharges. Whether they represent subcortical phenomena or restricted-field cortical discharges is unclear. Large-amplitude jerks may have EEG correlates, often very high-amplitude generalized spike-wave bursts, which are slower and less rhythmic than those associated with JME. The background activity of the EEG is slow, and this differentiates the conditions from idiopathic generalized epilepsy. Somatosensory evoked potentials (SEPs) are also abnormal, showing giant cortical responses.

Somatosensory or auditory reflex precipitation of seizures is more common in PME and light precipitation of seizures is more common in JME, with the exception of Unverricht-Lundborg disease, in which photosensitivity is marked and characteristic.

Neuroimaging can show atrophy, but there are no specific radiological features. A skin or muscle biopsy can be diagnostic for the PMEs.

The clinical course is well defined. Lafora body disease (LD) and neuronal ceroid lipofuscinosis (NCL) have an invariably fatal course, whereas others, such as Unverricht-Lundborg disease (EPM1), may be associated with a relatively normal life span if appropriate supportive treatment is implemented.

The present invention concerns also a pharmaceutical composition for preventing or treating progressive myoclonic epilepy comprising a therapeutically effective amount of an active compound brivaracetam and a pharmaceutically acceptable carrier.

Pharmaceutical compositions comprising the active compound can, for example, be administered orally or parenterally, i.e., intravenously, intramuscularly, subcutaneously or intrathecally.

Pharmaceutical compositions which can be used for oral administration can be solids or liquids and can, for example, be in the form of tablets, pills, dragees, gelatin capsules, solutions, syrups, and the like.

To this end, the active compound can be used mixed with an inert diluent or a non-toxic pharmaceutically acceptable vehicle such as starch or lactose, for example. Optionally, these pharmaceutical compositions can also contain a binder such as microcrystalline cellulose, gum tragacanth or gelatine, a disintegrant such as alginic acid, a lubricant such as magnesium stearate, a glidant such as colloidal silicon dioxide, a sweetener such as sucrose or saccharin, or colouring agents or a flavouring agent such as peppermint or methyl salicylate. They also comprise compositions which can release the active substance in a controlled manner. Pharmaceutical compositions which can be used for parenteral administration are in the pharmaceutical forms which are known for this mode of administration and are in the form of aqueous or oily solutions or suspensions generally contained in ampoules, disposable syringes, glass or plastics vials or infusion containers.

In addition to the active compound, these solutions or suspensions can optionally also contain a sterile diluent such as water for injection, a physiologic saline solution, oils, polyethylene glycols, glycerine, propylene glycol or other synthetic solvents, antibacterial agents such as benzyl alcohol, antioxidants such as ascorbic acid or sodium bisulphite, chelating agents such as ethylene diamine-tetra-acetic acid, buffers such as acetates, citrates or phosphates and agents for adjusting the osmolarity, such as sodium chloride or dextrose.

These pharmaceutical forms are prepared using methods which are routinely used by pharmacists. The percentage of active material in the pharmaceutical compositions can fall within a wide range of concentrations and depends on a variety of factors such as the patient's sex, age, weight and medical condition, as well as on the method of administration. Thus the quantity of active product in compositions for oral administration is at least 0.5% by weight and can be up to 80% by weight with respect to the composition weight. The terms "active material" and "active product" as used by the Applicant mean brivaracetam alone or combined with at least one other pharmaceutically active compound for use in these pathologies.

In compositions for parenteral administration, the quantity of active material present is at least 0.5% by weight and can be up to 33% by weight with respect to the composition weight. For the preferred parenteral compositions, the dosage unit is in the range 0.1 mg to 1500 mg of active product.

The efficacy of brivaracetam for preventing or treating disease states characterised by progressive myoclonic epilepies may be illustrated by the following pharmacological tests.

### Example 1

Brivaracetam is evaluated in a rat model of post-hypoxic myoclonus [Truong et al. (1994) Novel rat cardiac arrest model of post-hypoxic myoclonus. Mov. Disord. 9:201-206]. In this model, male Sprague-Dawley rats are subjected to mechanical-induced cardiac arrest for a short duration of time during which cerebral hypoxic insults are induced.

All surgerical procedures are performed under deep anaesthesia (ketamine 85 mg/kg ip and xylazine 15 mg/kg ip). The cardiac arrest is initiated and maintained for a duration of 8 minutes by mechanically obstructing all the major cardiac blood vessels including the aorta by hooking up the vessels with an L-shaped loop inserted into the rat body cavity. The arterial blood pressure is maintained at 0-10 mm Hg which leads to termination of the cerebral perfusion.

Resuscitation begins at 8 min following cardiac arrest by resuming manual cardiac compression and by IV administration of epinephrine (10 µg/kg) and sodium bicarbonate (4 mEq/kg).

These animals show jerking movements in response to auditory stimuli and other features of myoclonus that are similar to those observed in human condition. Myoclonic jerking movements in response to auditory stimulation is ranked according to their intensity. The auditory stimulus consists in 45 clicks of a metronome, each stimulus having a sound intensity of 96 dB with a duration of 40 ms at a frequency of 0.75 Hz. The involuntary muscle jerks in response to each stimulus are scored as follows: 0 = no jerks; 1 = ear twitch; 2 = ear and body jerk; 3 = ear, head and shoulder jerk; 4 = whole body jerk; and 5 = whole body jerk with jumping.

Antimyoclonic activity of Brivaracetam, administered ip at 10, 30, 100 and 300 mg/kg, is evaluated 48 hours after the cardiac arrest. The myoclonus score is recorded at 30 min interval over a period of 3 hours after dosing, in groups of 6 rats per dose.

Brivaracetam is tested according to the procedure described above,and is found active.

### Example 2

Brivaracetam is evaluated in a wide range of animal models of seizures and epilepsy. A short description of the different tests used in this evaluation is given below.

The effect of Brivaracetam against maximal electroshock seizures (MES) and pentylenetetrazol (PTZ) seizures is evaluated in male NMRI mice (20-30 g; N=10 per group). MES is induced through corneal electrodes delivering a current of 50 mA for a duration 0.2 s inducing tonic hind limb extension in 100% of saline-treated animals.

PTZ is administered at a dose of 89 mg/kg s.c. which represents a dose inducing clonic convulsions of all four extremities in 97% of saline-treated animals. The mice are pre-treated with either saline or different doses of Brivaracetam (i.p. adm., -30 min.) and the proportion of mice protected against seizures is recorded.

The effect of Brivaracetam on the threshold for DMCM-induced seizures is evaluated in male NMRI mice (25-35 g; N=10 per group). The threshold for induction of myoclonic jerks and clonic convulsions in all four extremities is determined by infusing a concentration of 0.5 mg/ml DMCM into the tail vein of unrestrained freely moving mice at a rate of 0.25 ml/min with an infusion pump and a maximum duration of infusion of 2 min. Saline or different doses of Brivaracetam are administered 30 min before DMCM infusion.

Brivaracetam is tested in male genetically sound-sensitive mice (16-28 g; N=10 per group) responding with wild running, clonic and tonic convulsions to an acoustic stimulation. Audiogenic seizures are induced by an acoustic stimulus (90 dB, 10 - 20 kHz) applied for 30 s. The mice are pre-treated with either saline or different doses of Brivaracetam (i.p. adm., -30 min.) and the proportion of mice protected against both clonic and tonic convulsions is used as the end point to assess anticonvulsant activity.

The effect of brivaracetam on kindled seizures is evaluated in male Sprague-Dawley rats (250-350 g) implanted with a bipolar stimulation / recording electrode into the right basolateral amygdala [AP-2.3, L-4.8, V-8.5 (Paxinos and Watson, 1982)], under pentobarbital anaesthesia. Kindling is induced by once daily stimulation, 5 days per week, with 500 µA monophasic square wave pulses, 50 Hz for 1 s (Löscher et al., 1986). The animals are considered kindled after the appearance of at least 10 consecutive stage 4 or 5 seizures according to the scale of Racine (Racine, 1972). Fully kindled rats (N=8 per group) are stimulated once with the same stimulation parameters as above after administration of saline (i.p., -60 min).

This procedure is repeated two days later, after pre-treatment with either saline or different doses of Brivaracetam (i.p., -60 min).

The behavioural effect of stimulation is graded according to the scale of Racine and the proportion of rats protected against generalised motor seizures (stage < 3) is recorded.

Male rats from the Genetic Absence Epilepsy Rat Strain are implanted with four platinum electrodes into the left and right frontal cortex and left and right occipital cortex, under pentobarbital anaesthesia. Cortical, spontaneous spike-and-wave discharges (SWDs) are recorded bilaterally from the rats placed into Plexiglass boxes, and prevented for falling asleep by gentle sensory stimulation. After a 20-min habituation period, the rats are injected i.p. with either saline or different doses of Brivaracetam and the EEG recorded continuously over consecutive 20-min intervals up to 120 min. The cumulative duration of the SWDs is calculated.

Brivaracetam produces a potent protection against different seizure types in both mice and rats (Table 1 and 2).

**Table 1: Anticonvulsant profile of Brivaracetam in different animal models of seizures and epilepsy**

| Model | Seizure parameter | Protective effect ED₅₀ value; mg/kg i.p. |
|---|---|---|
| MES | Tonic hind limb extension | 113 (89 - 136) |
| PTZ in mice | Clonic convulsions | 30 (10-87) |
| Audiogenic seizures in mice | Clonic convulsions | 2.4 (1.4-4.0) |
| | Tonic convulsions | 1.4 (0.9-2.1) |
| Amygdala kindling in rats | Secondary generalised seizures | 44 (24-84) |
| Genetic Absence Epilepsy Rats from Strasbourg | Duration of spike-and-wave discharges over 2 hours | 2.6 (0.85 - 8.1) |

| | | |
|---|---|---|
| Values in parentheses are the 95% confidence interval. | | |

**Table 2: Effect of Brivaracetam on the threshold for DMCM-induced seizures in mice**

| Brivaracetam (mg/kg IP) | First myoclonic jerks | | Clonic convulsion | |
|---|---|---|---|---|
| | DMCM threshold dose (mg/kg) | | DMCM threshold dose (mg/kg) | |
| 0 | 0.99 ± 0.19 | | 2.29 ± 0.74 | |
| 2.1 | 1.05 ± 0.14 | +6% | 3.34 ± 0.93 | +46% |
| 6.8 | 1.10 ± 0.16 | +11% | 4.68 ± 2.21 | +104% |
| 21.2 | 1.25 ± 0.14 | +26% | 6.47 ± 2.82 * | +183% |
| 67.9 | 1.31 ± 0.17 * | +32% | 6.46 ± 3.16 * | +182% |
| 212.3 | 1.35 ± 0.17 * | +36% | 7.16 ± 2.98 * | +213% |

| | | | | |
|---|---|---|---|---|
| Values given are means ± S.D. * : P<0.05 Kruskall Wallis followed by a post hoc Dunn's multiple comparison test vs control group All together these results indicate a broad spectrum anticonvulsant profile of Brivaracetam against different seizure types in rodents including myoclonic jerks, clonic and tonic seizures, partial seizures with secondary generalisation and generalised absence seizures. | | | | |

### Example 3

The aim of the placebo-controlled, single blind, multi-centre study is to explore the photoparoxysmal EEG (Electroencephalogram) response (PPR) in photosensitive epileptic subjects after one single oral dose (10, 20, 40 and 80 mg) of brivaracetam (BRV).

In the study, nineteen Caucasian subjects with photosensitive epilepsy (15 females, 4 males) with stable PPR in at least one eye condition (eyes open, eyes closed, eye closure) are enrolled. During a 3-day period, subjects underwent standardized intermittent photic stimulation (IPS) at different frequencies to define the standard photosensitivity range (SPR) in frequency steps per subject at fixed time points over the day: predose (-0.5 h) and postdose (1, 2, 4, 6, 8, 24, 28, 32, 48, and 72 h). After the placebo baseline day (day 1), single oral doses of BRV are given, starting at 80 mg, with subsequent lowering or increasing of the dose depending on the results per 4 subjects. Since positive results with the first dosage of 80 mg are observed, this dosage is successively reduced down to 10 mg.

A total of 18 subjects are evaluable. One subject appeared to be no longer sensitive on the baseline day and is therefore not evaluable. Under placebo administration almost no SPR changes from pre-dose in "eye-closure" condition are observed. Important SPR changes or even complete abolishment of the photosensitivity windows are observed after BRV administration at all tested doses.

All the single doses (10, 20, 40, and 80 mg of BRV) administered in the present study are effective in reducing (94%) or even abolishing the photoparoxysmal EEG response in photosensitive epileptic subjects (78%). All doses are safe and well tolerated. A clear dose-response is not identifiable, as both at 20mg and at 80 mg the EEG response in all subjects is abolished, with only one patient in the 20 mg group unresponsive due to his lack of EEG response in the pre-administration run.

Descriptive statistics (N, Median (range)) for time to first response and duration of response in "eye-closure" condition are given in the Table 3 below. Response is defined as a SPR change of at least 3 frequency steps. It is important to note the duration of response especially in the 80 mg group, which lasts well beyond the expected effect as based on the half-life of the compound of ~7.7 hours.

**Table 3: Time to first response and duration of response in "eye-closure" condition**

| Parameter | Drug | Dose | N^{(a)} | Median (Range) |
|---|---|---|---|---|
| Time to first response (h) | Placebo | 10 mg | 1 | 0.5 (0.5; 0.5) |
| | | 20 mg | 2 | 4.3 (0.5; 8.0) |
| | | 40 mg | 4 | 1.5 (0.5; 4.0) |
| | | 80 mg | 1 | 4.0 (4.0; 4.0) |
| | Brivaracetam | 10 mg | 4 | 0.5 (0.5; 1.0) |
| | | 20 mg | 4 | 0.5 (0.5; 1.0) |
| | | 40 mg | 5 | 0.5 (0.5; 1.0) |
| | | 80 mg | 4 | 0.5 (0.5; 0.5) |
| Duration of response (h) | Placebo | 10 mg | 1 | 1.5(1.5; 1.5) |
| | | 20 mg | 2 | 0.0 (0.0; 0.0) |
| | | 40 mg | 4 | 0.0 (0.0; 0.5) |
| | | 80 mg | 1 | 0.0 (0.0; 0.0) |
| | Brivaracetam | 10 mg | 4 | 29.3 (7.5; 31.5) |
| | | 20 mg | 4 | 27.5 (23.0; 31.5) |
| | | 40 mg | 5 | 27.0 (23.5; 47.5) |
| | | 80 mg | 4 | 59.5 (27.5; 71.5) |

| | | | | |
|---|---|---|---|---|
| (a) number of subjects who have a response. | | | | |

Median time to first response is 0.5 hours for all doses, and median duration of response is approximately 28 hours after 10, 20, or 40 mg BRV. The median duration is longer after 80 mg BRV (59.5 h). Time to maximal reduction in SPR is dose related: 1.5, 1.0, 1.0, and 0.5 hours, respectively, for 10, 20, 40, and 80 mg BRV. Plasma levels of brivaracetam (BRV) reach a peak at approximately 2.0 hours. T1/2 is about 8.5 hours. Effect of BRV is still frequently observable with BRV plasma levels below the lower level of quantification.

BRV preclinical data show a high efficacy against epilepsy, which, translated into therapeutic effects in humans, means a great reduction in seizure frequency and a higher number of responders and seizure-free patients in refractory epileptic patients.

Additionally, BRV shows strong efficacy in humans in a photoparoxysmal EEG response study in patients with photosensitive epilepsy. As shown above, BRV has abolished or reduced epileptiform discharges on the EEG following photic stimulation in patients at all tested doses (10, 20, 40, 80 mg single dose). In the same model, a single oral dose of 20 and 80 mg BRV led to the complete suppression of epileptiform discharges in the EEG of all treated and evaluable patients. However, a single dose of 10 mg of BRV still led to an abolishment of response in three out of four patients, and a reduction in EEG response in the fourth patient.

These results are considered relevant for a potential efficacy in patients with EPM1 (Unverricht-Lundborg disease) and LD (Lafora Disease), due to the photosensitivity of these patients. Knowing that precipitation of myoclonic jerks by intermittent photic stimulation is a characteristic feature of a major part of PME patient, the PPR results can be considered as potentially predictive for the efficacy of BRV in the treatment of these indications.

The preclinical profile shows a high potency and efficacy of BRV, a symptomatic effect on the myoclonus in EPM1 is shown, which may potentially extend to other PMEs with a predominantly myoclonic phenotype. Additionally, the potential for efficacy in the control of the generalized seizures present in the disease may provide the possibility for patients to reduce the number and dose of concomitant, less tolerable medications or even to revert to a monotherapy regimen.

## Claims

1. Use of (2S)-2-[(4R)-2-oxo-4-propylpyrrolidinyl]butanamide for the manufacture of a medicament for preventing or treating a disease state **characterised by** progressive myoclonic epilepsies.

2. Use according to claim 1 wherein disease states **characterised by** progressive myoclonic epilepsies are selected from Unverricht-Lundborg disease, Lafora disease, Myoclonic epilepsy with ragged red fibres, neuronal ceroid lipofuscinosis, sialidoses, and dentatorubral-pallidoluysian atrophy.

3. Use according to claim 1 wherein disease state is Unverricht-Lundborg disease.

4. A pharmaceutical composition for preventing or treating a disease state **characterised by** progressive myoclonic epilepsies comprising a therapeutically efective amount of (2S)-2-[(4R)-2-oxo-4-propylpyrrolidinyl]butanamide, and a pharmaceutically acceptable carrier.
